# EUROPEAN PATENT SPECIFICATION

(11) **EP 1 723 897 B1**
(45) Date of publication and mention of the grant of the patent: **28.11.2012**
(21) Application number: 05719838.4
(22) Date of filing: 02.03.2005
(51) Int. Cl.: G02B 23/24, A61B 1/005

(54) **ENDOSCOPE SYSTEM, ENDOSCOPE DEVICE, AND IMAGE PROCESSING DEVICE**
ENDOSKOPSYSTEM; ENDOSKOPVORRICHTUNG UND BILDDARSTELLUNGSVERFAHREN
SYSTEME ENDOSCOPE, DISPOSITIF ENDOSCOPE ET DISPOSITIF DE TRAITEMENT D'IMAGE

(30) Priority: 11.03.2004 JP 2004069374
(43) Date of publication of application: 22.11.2006
(73) Proprietor: Olympus Corporation, Tokyo 151-0072 (JP)
(72) Inventor: MIYAGI, Masaaki, 1920032 (JP); TAKASE, Seisuke, 1920916 (JP); MORIYAMA, Hiroki, 1960032 (JP)
(74) Representative: von Hellfeld, Axel
(86) International application number: PCT/JP2005/003523
(87) International publication number: WO 2005/087080

(56) References cited:
- EP-A1- 0 228 493
- DE-A1- 1 964 603
- JP-A- 6 181 885
- JP-A- 9 005 643
- JP-A- 2001 340 292
- JP-A- 2002 191 547
- JP-A- 2003 334 163
- US-A- 5 885 208

## Description

### TECHNICAL FIELD

The present invention relates to an endoscope system that allows endoscopes to observe a same observation area even if a range of bending motion of any endoscope is restricted with respect to others. Here, the range of bending motion is restricted with respect to the others since there are various outside diameters available for an insertion portion of the endoscope. Further, there are various outside diameters for the insertion portion since the outside diameter of the insertion portion corresponds to a function of an endoscope.

### BACKGROUND ART

Conventionally, an endoscope apparatus for observing body tissue inside a body cavity while inserting an insertion portion into the body cavity has been used in a medical field. Here, the insertion portion has an observation optical system at a distal end thereof. Further, an endoscope apparatus for observing a pipe interior, a narrow path interior, and the like has been used in an industrial field.

The endoscope is required to have a thin insertion portion. However, an outside diameter of the insertion portion depends on a function of the endoscope. For example, when the insertion portion only has a function for observing body tissue inside the body cavity by the endoscope, the outside diameter of the insertion portion can be thinned down since the insertion portion includes a light guide for guiding an illuminating light to an illuminating lens provided on an illumination window, an image guide for guiding a light of an observation area from the objective lens provided on an observation window, and a signal cable. The image guide may be replaced with a solid-state imaging sensor that is provided at a focal point of an objective lens. Further, when a function of the insertion portion is upgraded by adding additional components such as an insertion channel into which a forceps is inserted for extraction of the body tissue, an air and water supplying channel for supplying air and water to the body tissue, and an air and water aspiration channel for aspirating air and water, to the insertion portion in addition to the light guide, the image guide, and the signal cable, the outside diameter of the insertion portion is thickened. Similarly, when the function of the insertion portion is upgraded by increasing number of pixels of the solid-state imaging sensor, the outside diameter of the insertion portion is also increased.

When a medical endoscope is employed for the observation of an area inside a body cavity, the observed area may have a different shape and inner diameter. Consequently, an endoscope having an insertion portion with an outside diameter appropriate for the observation target area is used. However, even when one specific observation target area is observed, the outside diameter of the insertion portion of employable endoscopes may still depend on the function of the endoscope as described above. For example, the endoscope that can clearly observe the observation area in detail and perform many treatments has a thick insertion portion.

A bendable portion that bends a distal end portion upward, downward, leftward, and rightward along a shape of the observation area is provided at a distal end side of the insertion portion of the endoscope. A range of bending motion of the bendable portion depends on the outside diameter of the insertion portion and an inside diameter of a hollow organ in which the insertion portion is placed. That is to say, when the insertion portion is inserted into the hollow organ, which is the observation area, and when a sufficient space is maintained between the outer periphery of the insertion portion and an inner wall of the hollow organ, the range of bending motion of the bendable portion is wide. On the other hand, when the sufficient space is not maintained between the outer periphery of the insertion portion and the inner wall of the hollow organ, a distal end and a side face of the insertion portion is brought into contact with the inner wall, so that the range of bending motion of the bendable portion is narrow. Consequently, when a viewing angle of an objective lens provided on an observation window at a distal end of the insertion portion that can maintain the sufficient space just mentioned is the same as that of an objective lens provided on an observation window at a distal end of the insertion portion that cannot maintain the sufficient space just mentioned, the range of bending motions of the insertion portions differ from each other due to the difference in the outside dimensions of the insertion portions. Hence, an angle of view of the observation area that is observed through the objective lens of the insertion portion maintaining the sufficient space differs from that of the observation portion observed through the objective lens of the insertion portion that does not maintain the sufficient space.

As described above, the angle of view of the observation area that can be observed from the observation window depends on the difference in the outside diameter of the insertion portion and the inside diameter of the observation area into which the insertion portion is inserted. Hence, an endoscope that is capable of changing the angle of view of the observation by shifting an objective lens group provided on the observation window along a direction of an optical axis thereof has been proposed (for example, see Patent Document 1).
Patent Document 1: Japanese Patent Application Laid-Open No. 2001-258823 (page 5, FIG. 10, FIG. 11)
EP 0 228 493 discloses a searching and measuring endoscope employing two image-transmitting systems in a common housing. DE 1 964 603 and US 5,885,208 disclose endoscope systems comprising two endoscopes.

### DISCLOSURE OF INVENTION

### PROBLEM TO BE SOLVED BY THE INVENTION

The conventional endoscope, when upgraded, has a thick outside diameter of the insertion portion. On the other hand, when an observation space with a predetermined inside diameter, such as a large intestine, is observed by the endoscope by inserting the thin insertion portion that is formed to have comparatively thin diameter and bending the thin insertion portion, the range of bending motion of the bendable portion becomes comparatively wide since a comparatively wide space can be maintained between an outside of the thin insertion portion and an inner wall of the large intestine. On the other hand, the range of bending motion of the bendable portion of the endoscope with a comparatively thick insertion portion with respect to the thin insertion portion described above becomes narrow since the space between the outside of the endoscope with the thick insertion portion and the inner wall of the large intestine becomes narrow.

As described above, since the range of bending motion of the insertion portion is restricted due to a dimension of the space between the outside diameter of the insertion portion and the inside dimension of the observation space, there exists an endoscope as proposed in Patent Document 1 that maintains the angle of view of the observation. The endoscope maintains the angle of view of the observation even if the range of bending motion is narrow, by having a changeable angle of view. Here, the angle of view of the endoscope can be changed by shifting the objective lens group that constitutes the observation optical system provided on the distal end of the insertion portion in the direction of the optical axis thereof. Consequently, by providing an objective lens shifting function that allows adjustment of the angle of view of the observation, the endoscope is upgraded. Therefore, the upgrading is a factor in thickening the insertion portion.

On the other hand, when an observation space such as the large intestine whose inside diameter is comparatively uniform and which has plural folds is observed by an endoscope, the endoscope with a thick insertion portion having a function capable of treating a diseased part of body tissue is used. Hence, it is desirable to provide an endoscope system that allows for an observation of even a backside of the fold at the observation of the inside of the large intestine while the range of bending motion of the insertion portion thereof is restricted due to the inner wall of the large intestine.

The present invention is provided in order to alleviate the above inconveniences. Hence, an object of the present invention is to provide an endoscope system that is capable of maintaining an angle of view of an observation that is greater than or equal to an angle of view of an endoscope having a thin insertion portion, when employing an endoscope having a comparatively thick insertion portion with respect to an observation space.

### MEANS FOR SOLVING PROBLEM

The invention is defined in claim 1.

An endoscope system according to the present invention has plural endoscopes that are used with one portion thereof inserted into an observation space and includes a first endoscope having an insertion portion including a bendable portion that is bendable over a predetermined range of bending motion in a predetermined observation space, and a distal end portion including an observation optical system with a predetermined viewing angle, the distal end portion changing a direction of an optical axis thereof corresponding to bending of the bendable portion; and a second endoscope having an insertion portion including a bendable portion that is bendable over a range of bending motion in the predetermined observation space, the range of bending motion is restricted in a region narrower than a range of bending motion of the first endoscope, and a distal end portion including an observation optical system with a predetermined viewing angle wider than the viewing angle of the first endoscope, the distal end portion changing a direction of an optical axis thereof corresponding to bending of the bendable portion.

In the endoscope system according to the present invention, the viewing angle of the second endoscope is wider than or equal to the viewing angle of the first endoscope when a bending angle of the first endoscope is the same as a bending angle of the second endoscope.

In the endoscope system according to the present invention, an outside diameter of the insertion portion of the second endoscope is larger than an outside diameter of the insertion portion of the first endoscope.

An endoscope apparatus for the system according to the present invention has an observation optical system for observing a region inside a subject body and a bendable portion that is bent according to a bending manipulation, wherein the observation optical system has a viewing angle wider than a viewing angle of another endoscope apparatus whose bendable portion has a wider range of bending motion than a range of bending motion of the bendable portion of the endoscope apparatus, with respect to an identical region inside the subject body.

In the endoscope apparatus according to the present invention, the endoscope apparatus is connectable to a device which functions to operate an endoscope that is identical to the another endoscope.

An endoscope apparatus according to the present invention is used with one portion thereof inserted into an observation target and includes a bendable portion that is bendable over a predetermined range of bending motion in a predetermined observation space; and
a distal end portion including an observation optical system with a predetermined viewing angle, the observation optical system changing a direction of an optical axis corresponding to a bending manipulation of the bendable portion, wherein the viewing angle of the observation optical system is determined based on the range of bending motion of the bendable portion, or the range of bending motion of the bendable portion is determined based on the viewing angle of the observation optical system.

In the endoscope apparatus according to the present invention, the viewing angle and the range of bending motion are determined based on a preliminarily determined observation region.

The system according to the invention furthermore comprises an image processing apparatus for which processes observation image data acquired by plural observation optical systems with different viewing angle and includes a display size determining unit that determines a display size of the observation image data based on the viewing angle of the observation optical system that generates an observation image to be displayed so that a difference of display size on a display screen of a region corresponding to a unit viewing angle between observation image data acquired by the observation optical system and observation image data acquired by another observation optical system is reduced; and a size conversion processing unit that performs a process in which the observation image data is electronically enlarged and/or contracted so that the observation image data has a display size determined by the display size determining unit.

In the image processing unit according to the present invention, the observation image data is acquired by a first endoscope having an observation optical system with a predetermined viewing angle and by a second endoscope having an observation optical system with a wider viewing angle than the viewing angle of the first endoscope, and the display size determining unit determines the display size so that the display size of the observation image data acquired by the second endoscope becomes relatively large compared to the display size of the observation image data acquired by the first endoscope.

In the image processing apparatus according to the present invention, the display size determining unit determines the display size of the observation image data so that the display size of a region corresponding to a unit viewing angle on the display screen becomes substantially the same independently of the viewing angle of the observation optical system.

In the image processing apparatus according to the present invention, the size conversion processing unit further clips out only a midsection of the observation image data in order to realize a preliminarily determined display size on the display screen when an enlarging process is performed to the observation image data.

### EFFECT OF THE INVENTION

The endoscope system and the endoscope apparatus of the present invention can perform an observation and a treatment inside a restricted observation space using a high-performance endoscope having a thick insertion portion, by securing an equal degree of field of view to the field of view of an endoscope having a thin insertion portion, even though a range of bending motion of the thick insertion portion is restricted by the observation space, whereby the endoscope system and the endoscope apparatus of the present invention can provide an efficient endoscopic observation.

### BRIEF DESCRIPTION OF DRAWINGS

FIG. 1 is a block diagram showing an overall configuration of an embodiment of an endoscope system according to the present invention;
FIG. 2 is an explanatory view of a range of bending motion and a viewing angle of an insertion portion of an endoscope that is used for the endoscope system according to the present invention;
FIG. 3 shows a relationship between a bending angle and the viewing angle of the insertion portion of the endoscope in the endoscope system according to the present invention;
FIG. 4 is an explanatory view illustrating a manner of displaying an observation image on a monitor in the endoscope system according to the present invention;
FIG. 5 is an explanatory view explaining a display of a zoomed observation image on the monitor in the endoscope system according to the present invention; and
FIG. 6 is an explanatory view illustrating a configuration of an endoscope apparatus in the endoscope system according to the present invention.

### EXPLANATIONS OF LETTERS OR NUMERALS

- 11: First endoscope
- 12: Second endoscope
- 13: Camera control unit (CCU, connecting device)
- 14: Monitor
- 15, 31: Objective lens
- 16, 32: Solid-state imaging sensor (CCD)
- 17, 33: Correlated double sampling circuit (CDS circuit)
- 18, 34: Analog/digital conversion circuit (A/D circuit)
- 20: Viewing angle deriving unit
- 21: Separation processing circuit (S/P circuit)
- 22: Digital signal processing circuit (DSP circuit)
- 23: Textual information superposing circuit
- 24: Textual information inputting circuit
- 25: Digital/analog conversion circuit (D/A circuit)
- 26: Image displaying signal circuit
- 27: Reference signal generator circuit (SSG circuit)
- 28: Timing signal generator circuit (T/G circuit)
- 29: Display size determining unit

### BEST MODE(S) FOR CARRYING OUT THE INVENTION

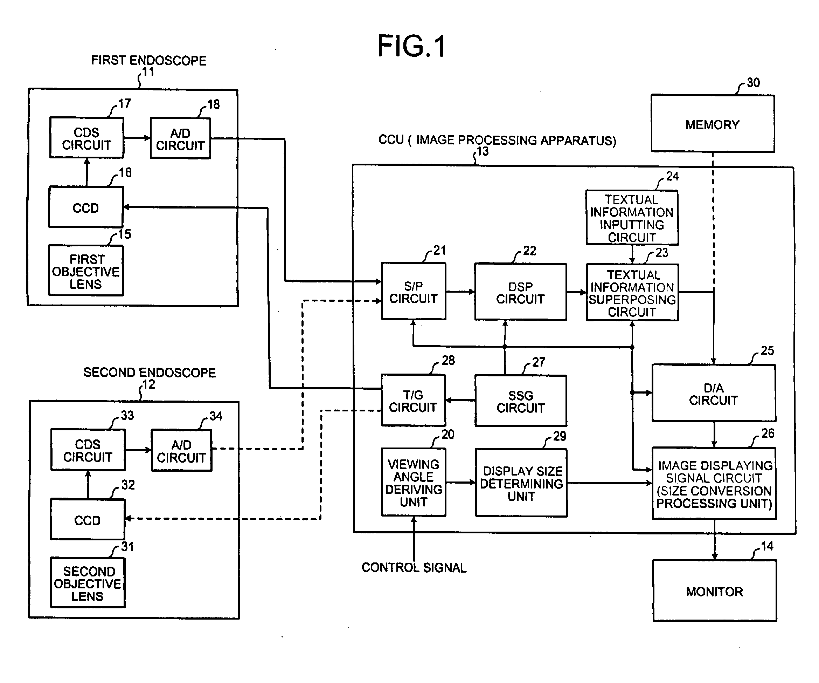

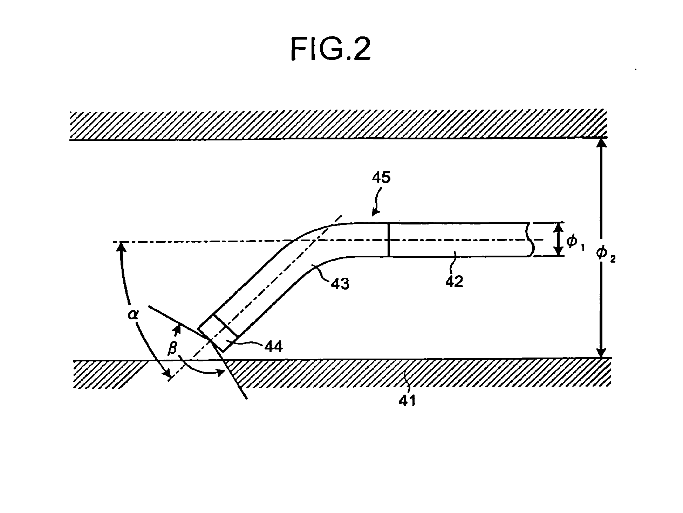

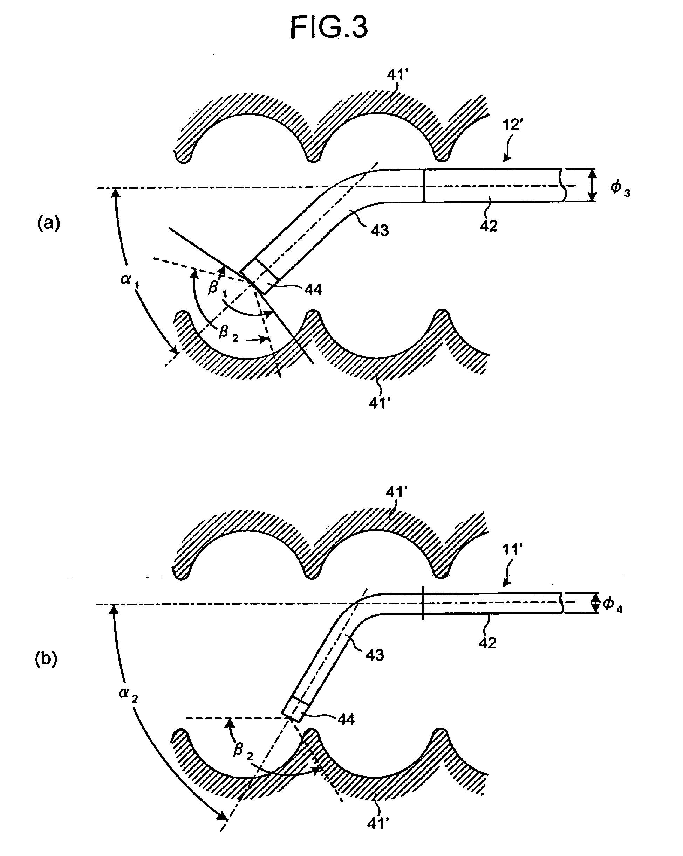

Embodiments of an endoscope system of the present invention will be explained below with reference to the accompanying drawings. An embodiment of the endoscope system according to the present invention is explained with reference to FIGS. 1 to 3. FIG. 1 is a block diagram showing an overall configuration of an embodiment of an endoscope system according to the present invention. FIG. 2 is an explanatory view illustrating a range of bending motion and a viewing angle of an insertion portion of an endoscope that is used for the endoscope system according to the present invention. FIG. 3 shows a relationship between a bending angle and the viewing angle of the insertion portion of the endoscope in the endoscope system according to the present invention. FIG. 3(a) is an explanatory view of the bending angle and the viewing angle of a thick insertion portion. FIG. 3(b) is an explanatory view of the bending angle and the viewing angle of a thin insertion portion.

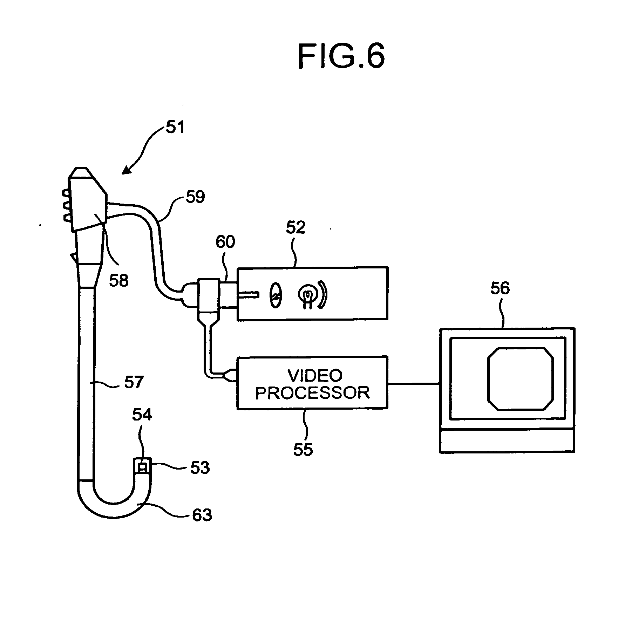

First, a schematic configuration of an endoscope apparatus that is used for the endoscope system according to the present invention is explained with reference to FIG. 6. The endoscope apparatus includes an endoscope 51, a light source 52, a video processor 55, and a monitor 56. The endoscope 51 has a distal end portion 53, a bendable portion 63, a flexible portion 57, a manipulating portion 58, a universal cord 59, and an endoscope connector 60.

An illumination window, an observation window, a forceps channel opening, an air and water supplying channel, and the like that are not shown are provided at the distal end portion 53 of the endoscope 51. A solid-state imaging sensor 54 that captures an image of an observation area is provided at the observation window of the distal end portion 53. The bendable portion 63 is provided at a rear end of the distal end portion 53. Plural bending blocks are arranged in the bendable portion 63, and the bendable portion 63 is bent upward, downward, leftward, and rightward by a bending wire extending from a bending knob provided on the manipulating portion 58. The flexible portion 57 is installed at a rear end of the bendable portion 63. The flexible portion 57 is formed from a flexible member in an elongated shape.

A light guide, a signal cable, a forceps channel, and an air and water supplying channel are arranged in the distal end portion 53, the bendable portion 63, and the flexible portion 57. A distal end of the light guide is arranged at the illumination window of the distal end portion 53. A distal end of the signal cable is connected to the solid-state imaging sensor 54 that is provided at the observation window. A distal end of the forceps channel is arranged at the forceps channel opening of the distal end portion 53. The air and water supplying channel is arranged at an air and water supplying channel opening of the distal end portion 53.

A proximal end of the light guide is connected to the light source 52 through the universal cord 59 and the endoscope connector 60 from the manipulating portion 58. A proximal end of the signal cable is connected to the video processor 55 through the universal cord 59 and the endoscope connector 60 from the manipulating portion 58. A distal end of the forceps channel is connected to a forceps inserting hole provided at the manipulating portion 58. A proximal end of the air and water supplying channel is connected to an air and water supplying channel venting cap provided at the manipulating portion 58, and air and water are supplied by an air and water supplying switch provided on the manipulating portion 58.

The light source 52 has an illuminating lamp and a lighting control circuit of the illuminating lamp, and projects an illuminating light onto the proximal end of the light guide of the endoscope connector 60. The video processor 55 drives the solid-state imaging sensor 54 provided at the distal end portion 53 as well as takes in a generated imaging signal of an image of an observation area and performs a predetermined signal processing with respect to the imaging signal to generate a standard image signal. The monitor 56 reproduces and displays the image of the observation area captured by the solid-state imaging sensor 54 based on the standard image signal generated at the video processor 55. Here, for example, name, age, and gender of a patient, and date and time of the observation by the endoscope are simultaneously displayed on the monitor 56 with the image of the observation area.

A configuration of the endoscope system according to the present invention used for the endoscope apparatus described above is explained with reference to FIG. 1. The endoscope system includes a first endoscope 11 and a second endoscope 12 that correspond to the endoscope 51; a camera control unit (hereinafter referred to as CCU) that functions as an example of an image processing apparatus in the claims, and corresponds to the video processor 55; and a monitor 14 that corresponds to the monitor 56. Here, the light source that generates the illuminating light projected from the first and the second endoscopes 11 and 12 onto the observation area is not shown.

The first endoscope 11 has a general viewing angle of, for example, 140v. The first endoscope 11 includes a first objective lens 15 that functions as an observation optical system in the first endoscope 11; a solid-state imaging sensor (hereinafter referred to as charge-coupled device (CCD)) 16 that is arranged at an imaging position of the first objective lens 15 and captures the image of the observation area; a correlated double sampling (CDS) circuit 17 that performs correlated double sampling processing on the imaging signal generated by the CCD 16; and an analog/digital conversion circuit (hereinafter referred to as A/D circuit) 18 that converts an analog imaging signal processed by the CDS circuit 17 to a digital imaging signal.

The second endoscope 12 has a viewing angle larger than that of the objective lens 15 of the first endoscope 11 and, for example, the viewing angle thereof is 170°. The second endoscope 12 includes a second objective lens 31 that functions as the observation optical system in the second endoscope 12; a solid-state imaging sensor (hereinafter referred to as CCD) 32 that is arranged at an imaging position of the second objective lens 32 and captures the image of the observation area; a CDS circuit 33 that performs correlated double sampling processing on the imaging signal generated by the CCD 32; and an analog/digital conversion circuit (hereinafter referred to as A/D circuit) 34 that converts an analog imaging signal processed at the CDS circuit 33 to a digital imaging signal.

The CCU 13 includes a separation processing circuit (hereinafter referred to as S/P circuit) 21; a digital signal processing circuit (hereinafter referred to as DSP circuit) 22; a textual information superposing circuit 23; a textual information inputting circuit 24; a digital/analog signal conversion circuit (hereinafter referred to as D/A circuit) 25; an image displaying signal circuit 26 that functions as an example of the size conversion processing unit in claims; a reference signal generator circuit (hereinafter referred to as SSG circuit) 27; a timing signal generator circuit (hereinafter referred to as T/G circuit) 28; and a display size determining unit 29 that determines a display size of an observation image data when the observation image data is displayed on the monitor 14 based on a viewing angle deriving unit 20 and the derived viewing angle. Here, the viewing angle deriving unit 20 derives the viewing angle of the observation optical system that captures the observation image data based on a predetermined control signal.

The S/P circuit 21 separates a luminance signal, a color signal, and the like, of the digital imaging signal from the A/D circuit 18 of the first endoscope 11 or of the digital imaging signal from the A/D circuit 34 of the second endoscope 12. The DSP 22 performs a predetermined digital signal processing as well as performs a correction processes such as white balance correction and γ correction with respect to the luminance signal and the color signal separated by the S/P circuit 21. Then, the DSP 22 generates a digital endoscope image signal.

The textual information superposing circuit 23 superposes textual information signals indicating endoscope observation information such as, name, age, and gender of a patient and date and time of the endoscope observation, on the digital endoscope image signal that is a signal processed by the DSP circuit 22. The textual information signal to be superposed by the textual information superposing circuit 23 is generated from the endoscope observation information that is input by an operator through a keyboard not shown at the textual information inputting circuit 24. The digital endoscope image signal that the textual information is superposed by the textual information superposing circuit 23 is converted to the observation image data in the D/A circuit 25, and then output to the image displaying signal circuit 26. Here, the digital endoscope image signal that the generated textual information signal is superposed by the textual information superposing circuit 23 is stored in a memory 30 that is detachably attached to the CCU 13.

The viewing angle deriving unit 20 derives the viewing angle of the observation optical system that is used for capturing the input observation image data. Specifically, the viewing angle deriving unit 20 derives the viewing angle of the observation image data input to the CCU 13, which is the image processing apparatus, based on, for example, a predetermined control signal input from outside. Here, the control signal that is input to the viewing angle deriving unit 20 may have a specific value of the viewing angle, or may contain information indicating which of the first endoscope 11 and the second endoscope 12 acquires the input observation image data. That is to say, the viewing angle deriving unit 20 may, for example, preliminarily recognize the viewing angle of the observation optical system (first objective lens 15) that is provided in the first endoscope 11 and the viewing angle of the observation optical system (second objective lens 31) that is provided in the second endoscope 12. Then, the viewing angle deriving unit 20 may derive a corresponding viewing angle by recognizing which of the endoscopes acquire the observation image data, based on the control signal.

Further, as a mechanism that generates the control signal, the keyboard and the like, for example, can be used as an input device. However, the first endoscope 11 and the second endoscope 12 may generate the control signal. That is to say, for example, the first endoscope 11 and the second endoscope 12 may further include a function to generate an identification signal to identify themselves, and the identification signal can be output to the CCU 13, which is the image processing apparatus, as the control signal while superposing the identification signal on the observation image data.

The display size determining unit 29 determines the display size of the observation image data to be displayed on the monitor 14 based on the viewing angle of the observation optical system. The viewing angle is derived by the viewing angle deriving unit 20. Specifically, plural observation optical systems capture the observation image data. Since the viewing angle is different from one observation optical system to another, a display area corresponding to one unit of the viewing angle (for example, the region on the observation image corresponding to a viewing angle of 1°) may vary when the observation image data is displayed on the monitor 14. The display size determining unit 29 determines the display size of the overall observation image data to alleviate or more preferably eliminate the difference between the display areas.

The image displaying signal circuit 26 functions as an example of the size conversion processing unit in the claims. Specifically, the image displaying signal circuit 26 converts the observation image data in analog format, which is supplied from the D/A circuit 25, to a standard image signal for displaying the observation image and the endoscope observation information on the monitor 14. When the conversion process is performed, the image displaying signal circuit 26 electronically enlarges and/or contracts the observation image data so that the observation image data obtains a display size, which is determined in the display size determining unit, on the display screen.

The SSG circuit 27 generates and outputs a reference signal that controls driving of the S/P circuit 21, the DSP circuit 22, the textual information superposing circuit 23, the D/A circuit 25, and the image displaying signal circuit 26. The T/G circuit 28 generates a timing signal to drive control each of the CCD 16 and 32 of the first and the second endoscopes 11 and 12 based on the reference signal from the SSG circuit 27.

Here, the first endoscope 11 has the first objective lens 15 with a general viewing angle of 140v. Further, the first endoscope 11 with general endoscopic functions has comparatively thin outside diameter of the insertion portion but not shown in the drawings. In comparison to the first endoscope 11, the second objective lens 31 of the second endoscope 12 has a wide viewing angle of 170° as described above. Further, the function of the second endoscope 12 is upgraded as an endoscope, and the outside diameter of the insertion portion of the second endoscope 12 is thicker than that of the first endoscope 11 but not shown in the drawings.

Further, the first endoscope 11 and the second endoscope 12 are connected to the CCU 13 through a connector and the like when necessary, or always connected to the CCU 13 through the connector, and the connection can be switched by a switch not shown.

Next, a relationship between the insertion portions of the first and the second endoscopes 11 and 12 and the observation space containing the observation area is explained with reference to FIG. 2. An insertion portion 45 of the first endoscope 11 or the second endoscope 12 has a distal end portion 44; a bendable portion 43; and a flexible portion 42, in this order from the distal end thereof. The first objective lens 15 and the CCD 16, or the second objective lens 31 and the CCD 32 are arranged at the distal end portion 44.

Let a bending angle that is formed by a direction of the optical axis of the distal end portion 44 when the bendable portion 43 is bent and by an axial direction of the flexible portion 42 be α, and let the viewing angle of the first objective lens 15 or the second objective lens 31 that is arranged at the distal end portion 44 be β. Further, let an outside diameter of the insertion portion 45 be φ1, and let an inside diameter of a hollow organ 41 into which the insertion portion 45 is inserted be φ2.

When the insertion portion 45 with the outside diameter φ₁ that is 1/4 of the inside diameter φ₂ of the hollow organ 41 (φ₁ = φ₂/4) is inserted into the hollow organ 41 and bent, the bending angle α when the distal end portion 44 and the flexible portion 42 are brought into contact with the inner wall of the hollow organ 41 becomes comparatively large.

On the other hand, when the insertion portion 45 with the outside diameter φ₁ that is 1/2 of the inside diameter φ₂ of the hollow organ 41 (φ₁ = φ₂/2) is inserted into the hollow organ 41 and bent, the bending angle α when the distal end portion 44 and the flexible portion 42 are brought into contact with the inner wall of the hollow organ 41 becomes comparatively small. That is to say, with respect to the hollow organ with the same inside diameter φ₂, the bending angle a is large for the insertion portion 45 with the outside diameter φ₁ that is thin (φ₂/4), and the bending angle a is small for the insertion portion 45 with the outside diameter φ₁ that is thick (φ2/2). Here, φ₂/4 = α > φ₂/2 = α.

Therefore, when the viewing angle P of the objective lens that is provided on the distal end portion 44 is the same for the thick insertion portion and the thin insertion portion, the bending angle α of the thick insertion portion 45 in which a difference between the outside diameter φ₁ of the insertion portion 45 and the inside diameter φ₂ of the hollow organ 41 is small becomes small, thus the observation angle of view becomes narrow. Further, the bending angle α of the thin insertion portion 45 in which a difference between the outside diameter φ₁ of the insertion portion 45 and the inside diameter φ₂ of the hollow organ 41 is large becomes large, thus the observation angle of view becomes wide.

As described above, when the insertion portion 45 with different outside diameter φ₁ is inserted into the hollow organ 41 with the same inside diameter φ₂ and bent, a difference in the bending angle α is caused. Consequently, a difference in the observation angle of view of the distal end portion 44 is caused. An influence due to the difference in the bending angle α due to the outside diameter φ₁ of the insertion portion 45 is minute when the hollow organ 41 having a straight pipe shape is observed. However, various hollow organs of a body, which is the observation target, have bent shape, as well as there are folds having complicated shapes on the inner wall thereof. Hence, when the outside diameter φ₁ of the insertion portion 45 is thick so that the bending angle α is small, the backside of the fold with respect to a direction of the insertion of the insertion portion 45 might not be observed.

A range of bending motion obtained at a time of observation of an interior of the hollow organ that has folds with complicated shapes is explained with reference to FIG. 3. Suppose that the observation is performed with the endoscopes 11 and 12 of the present invention shown in FIG. 1, and that the outside diameter φ₁ of the insertion portion 45 and the viewing angle β of the objective lens (15 and 31) are different in each endoscope.

FIG. 3(a) shows a state in which an insertion portion 12' of the second endoscope 12 is inserted into a large intestine 41' and is bent. The insertion portion 12' (hereinafter referred to as thick second insertion portion 12') has an outside diameter φ₃, and the maximum bending angle is α₁. FIG. 3(b) shows a state in which an insertion portion 11' of the first endoscope 11 is inserted into the large intestine 41' and is bent. The insertion portion 11' (hereinafter referred to as thin first insertion portion 11') has a thin outside diameter φ₄ (φ₄ < φ₃), and a maximum bending angle thereof is α₂ (α₂ > α₁).

Let the viewing angle of the objective lens 31 that is provided at the distal end portion 44 of the thick second insertion portion 12' and the viewing angle of the objective lens 15 that is provided at the distal end portion 44 of the thin first insertion portion 11' be a viewing angle β₂. Here, as shown in FIG. 3(a), an entire observation angle of view when the thick second insertion portion 12' is bent to have the maximum bending angle α₁ is (α₁ + β₂/2), in which 1/2 of the viewing angle β₂ of the objective lens 31 is added to the maximum bending angle α₁. On the other hand, as shown in FIG. 3(b), the entire observation angle of view when the thin first insertion portion 11' is bent to have the maximum bending angle of α₂ is (α₂ + β₂/2), in which 1/2 of the viewing angle β₂ of the objective lens 31 is added to the maximum bending angle α₂. Since the relation α₂ > α₁ is held between the bending angles α of the thin first insertion portion 11' and the thick second insertion portion 12', the relation (α₂ + β₂/2) > (α₁ + β₂/2) is held between the entire observation angles of view of the thin first insertion portion 11' and the thick second insertion portion 12'. The entire observation angle of view of the thick second insertion portion 12' is narrower, so that the backside of the fold of the large intestine 41' cannot be observed.

Therefore, the viewing angle P of the objective lens 31 that is provided at the distal end portion 44 of the thick second insertion portion 12' is set to have a wide viewing angle β₁ (β₁ > β₂). Consequently, the entire observation angle of view of the thick second insertion portion 12' becomes (α₁ + β₁/2), in which 1/2 of the wide viewing angle β₁ is added to the maximum bending angle α₁. As a result, the entire observation angle of view (α₁ + β₁/2) of the thick second insertion portion 12' becomes wider than the entire observation angle of view (α₁ + β₂/2) at when the objective lens 31 has the viewing angle of β₂, i.e., (α₁ + β₁/2) > (α₁ + β₂/2). Hence, the entire angle of view of the thick second insertion portion 12' can be set substantially the same as the entire observation angle (α₂ + β₂/2) of the thin first insertion portion 11', i.e., (α₁ + β₁/2) = (α₁ + β₂/2), so that the backside of the fold of the large intestine 41' can be observed.

That is to say, the maximum bending angle α₁ of the second endoscope 12 is restricted by the inner wall of the hollow organ since the outside diameter φ₂ of the insertion portion 12' becomes thick due to the upgrading of the function of the endoscope. Consequently, the observation angle of view becomes narrow. However, the entire observation angle of view can be widened by setting the objective lens 31 to have the wide viewing angle β₁.

In the first endoscope 11 that has the thin first insertion portion 11' with a narrow viewing angle β₂, the objective lens thereof can easily be manufactured in comparison to that of the second endoscope 12 that has the thick second insertion portion 12' with the wide viewing angle β₁. Further, a projecting region of the illuminating light illuminating the observation area is narrow so that the number of fiber optics configuring the light guide can be decreased. Hence, there is an advantage that a manufacturing cost of the endoscope can be reduced.

Further, although the endoscope system having plural endoscopes (first endoscope 11, second endoscope 12) is explained as an example in the present embodiment, a concept of the present embodiment is applicable to a structure of a single endoscope. Specifically, in the configuration of the single endoscope, a value corresponding to the range of bending motion of the bendable portion 43 or a value corresponding to the viewing angle of the observation optical system in a predetermined observation space is preferably determined in such a way that one of the values is determined based on the other.

Conventionally, specific values of the range of bending motion and the viewing angle of the observation optical system inside the observation space of the bendable portion 43 are each determined separately corresponding to the diameter of the insertion portion 45, a performance of the observation optical system, or the like. Consequently, the value of the observation angle of view that is determined based on the range of bending motion and the viewing angle might differ for each type of a product. Thus, when the plural endoscopes are simultaneously used to perform treatment and the like, there are problems that operability of the endoscope is decreased due to the difference in the observation angle of view. On the other hand, there is an advantage in the endoscope system of the present embodiment such that the observation angle of view suitable for the observation objective can be provided by relating the range of bending motion and the viewing angle to each other to determine the range of bending motion and the viewing angle. Further, by designing the endoscope system while relating the range of bending motion and the viewing angle to each other, the range of bending motion of the bendable portion can be restricted to a small range to have the predetermined observation angle of view when, for example, the observation optical system with the wide angle is used. Hence, there is an advantage that, for example, it is possible to design a large outside diameter of the insertion portion.

Next, an operation of the CCU 13 to which the first endoscope 11 and the second endoscope 12 are connected is explained. As described above, the observation image inside the subject body is captured by the first endoscope 11 and the second endoscope 12 each having a different viewing angle. Then, the predetermined signal processing is performed on the outputs of the first endoscope 11 and the second endoscope 12 by the S/P circuit 21, the DSP circuit 22, the textual information superposing circuit 23, the D/A circuit 25, and the like of the CCU 13, which is the image processing apparatus. Then, the observation image data in analog format is generated. On the other hand, the viewing angle deriving unit 20 derives the viewing angle of the observation optical system that acquires the observation image data based on the input control signal, and outputs the derived viewing angle with respect to the display size determining unit 29. The display size determining unit 29 changes the display size on the display screen of the monitor 14 of the observation image data based on the derived viewing angle.

Specifically, the display size determining unit 29 determines an appropriate display size of the observation image data so that the display size determining unit 29 alleviates or eliminate the difference between the display size of the region corresponding to the unit viewing angle of the observation image data that is acquired by the first endoscope 11 and the display size of the region corresponding to the unit viewing angle of the observation image data that is acquired by the second endoscope 12. For example, when the CCD 16 and 32 have the same number of pixels, the observation image data that is acquired at each of the first endoscope 11 and the second endoscope 12 are displayed on the display screen of the monitor 14 as the image with the display size which is the same to each other. When the viewing angles of the observation optical systems differ from each other as described above, the display sizes of the regions corresponding to the unit viewing angles differ from each other since the overall display sizes are the same to each others. Hence, the display size determining unit 29 determines the display size of the entire observation image data in such a way to alleviate or eliminate the difference of the display size of the region corresponding to the unit viewing angle.

Then, the image displaying signal circuit 26 that functions as the size conversion processing unit performs the electrical enlargement or contraction processing on the observation image data to display the observation image data on the display screen of the monitor 14 with the display size that is determined by the display size determining unit 29, while converting the observation image data in analog format that is output from the D/A circuit 25 to the standard image signal displayable on the monitor 14.

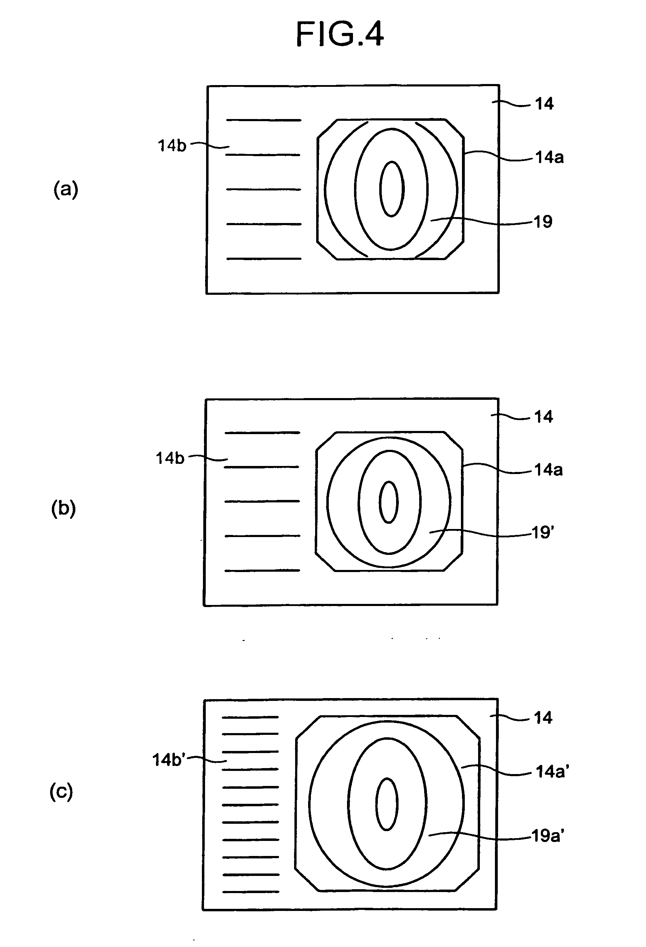

FIGS. 4(a) to 4(c) are schematic diagrams showing an example of the image that is displayed on the display screen of the monitor 14. As shown in FIGS. 4(a) to 4(c), an observation image displaying region (hereinafter referred to as image displaying region) 14a and an endoscope observation information displaying region (hereinafter referred to as information displaying region) 14b are formed on the display screen. In the image displaying region 14a, the endoscope images of the observation area that are captured by the CCD 16 and 32 of the endoscopes 11 and 12 are displayed. In the information display region 14b, the endoscope observation information such as the patient information, date and time of the observation, and the like that are superposed by the textual information superposing circuit 23 are displayed.

FIG. 4(a) is a schematic diagram showing a display example of the observation image data that is acquired by the first endoscope 11, and FIG. 4(b) is a schematic diagram showing a display example when the enlargement and the contraction processes are not performed with respect to the observation image data acquired by the second endoscope 12 that has the observation optical system with the viewing angle larger than the viewing angle of the observation optical system of the first endoscope 11. Here, in order to simplify the explanations, pieces of observation image data that correspond to the same target are used in examples that are shown in FIGS. 4(a) to 4(c). Further, the number of pixels, shape and dimension of a light receiving surface of the CCD 16 that is provided in the first endoscope 11 and of the CCD 32 that is provided in the second endoscope 12 are assumed to be equal.

It is apparent by comparing FIG. 4(a) and FIG. 4(b) that the display sizes of the observation target on the display screen of the monitor 14 differ from one another corresponding to the difference in the viewing angle, when the observation image data is output to the monitor 14 without performing the enlargement and the contraction processes with respect to the display size in the image displaying signal circuit 26. That is to say, when the number of pixels and the like of the CCD 16 and the CCD 32 are the same, the display sizes of the entire observation image data become the same to each other, independently of the difference in the viewing angle. Hence, the display size of the region corresponding to the unit viewing angle of the observation image data that is acquired by the second endoscope having the observation optical system with relatively wide viewing angle becomes small compared to the display size of the observation image data that is acquired by the first endoscope. In other words, even though the same object is captured, the display size of the imaging target on the display screen in the example of FIG. 4(b) that displays the observation image data acquired by the second endoscope becomes small compared to the display size in the example of FIG. 4(a) due to the difference in the viewing angle.

That is to say, the region of the observation image captured by the first endoscope is determined by the viewing angle of the first objective lens 15. Then, the observation image corresponding to the viewing angle is displayed on the image displaying region 14a on the display screen of the monitor 14 as the observation image 19 as shown in FIG. 4(a) while the endoscope observation information is displayed on the information displaying region 14b.

On the other hand, the observation image that is captured at the second endoscope 12 has the observation angle of view by the viewing angle of the second objective lens 31 that is wider compared to the observation angle of view of the first objective lens 15. Consequently, the observation image of the observation angle in the viewing angle of the second objective lens 31 is displayed on the image displaying region 14a of the monitor 14 as the observation image 19' as shown in FIG. 4(b) while the endoscope observation information is displayed on the information displaying region 14B.

That is to say, the observation image is displayed as the observation image 19' of the second endoscope 12 with respect to the observation image 19 of the first endoscope 1 that is displayed on the image displaying region 14a of the monitor 14. Here, the observation image that is displayed as the observation image 19' is captured at the viewing angle with the wider angle compared to the viewing angle of the first endoscope 11. As described above, although the observation image 19' of the second endoscope 12 that is displayed on the image displaying region 14a of the monitor 14 is displayed as an image corresponding to the wide viewing angle, each of the observation areas inside the observation angle of view is displayed small. Hence, there is a problem that the observation area might be difficult to be recognized. Further, there is a problem that the observation area might be missed out.

On the other hand, in the present embodiment, the observation image data that is acquired by the second endoscope 12 is displayed on the display screen as shown in FIG. 4(c) by the effect of the display size determining unit 29 and the image displaying signal circuit 26 described above. That is to say, the display size of the entire observation image data is enlarged in such a way that the observation image data that is acquired by the second endoscope comes to have substantially the same display size of the region corresponding to the unit viewing angle as that of the observation image data acquired by the first endoscope. As a result, the pieces of observation image data are displayed while display sizes on the display screen of the monitor 14 are equal to each other for the same target.

Specifically, as shown in FIG. 4(c), the observation image is enlarged as shown by the image displaying region 14a' of the monitor 14 while the information displaying region 14b' is contracted. Thus, by enlarging the image displaying region 14a on the display screen of the monitor 14 as the image displaying region 14a', the observation image 19a' that is displayed on the enlarged displaying region 14a' is also enlarged and displayed. Since the enlarged observation image 19a' can show the observation area larger, the observation area can be recognized to the same degree of details as FIG. 4(a), so that any miss out of the observation area can be dissolved. Here, deterioration of image quality due to the enlargement of the enlarged observation image 19a' can be reduced by increasing the number of pixels of the CCD 32 of the second endoscope 12 compared to the number of the pixels of the CCD 16 of the first endoscope 11.

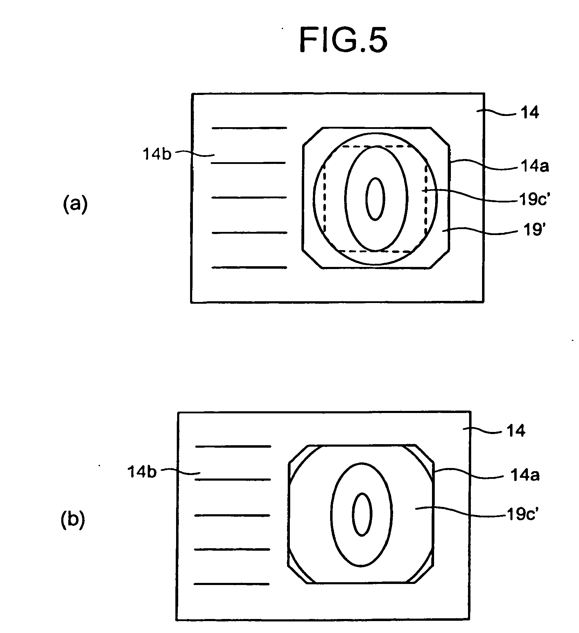

Next, a modification of the observation image data processing is explained with reference to FIG. 5. In the present modification, the image displaying signal circuit 26 adjusts the display size by clipping out a midsection of the observation image when the display size of the observation image data increases by a predetermined amount due to the enlargement of the observation image data to set the display size of the observation image data to the display size determined by the display size determining unit 29.

As described above, the wide angle observation image 19' that is captured by the second endoscope 12 having the second objective lens 31 with the wide angle is displayed on the image displaying region 14a of the monitor 14 as shown in FIG. 5(a). Hence, a wide observation region can be observed by the wide angle observation image 19' due to the second endoscope 12. Here, the wide observation image 19' is displayed on the image display region 14a of the monitor 14. However, it is preferred that each of the observation regions be observed with the wide observation angle of view.

Hence, in the present modification, the image displaying signal circuit 26, which is the size conversion processing unit, performs a predetermined process with respect to the observation image data in such a way that the observation image data has the display size that is determined by the display size determining unit 29. Then, by extracting the data corresponding to the midsection of the observation image and by cutting out the data corresponding to the surrounding sections, it is possible to maintain the size of the image displaying region 14a as shown in FIG. 5(b) as well as to display the central image section 19c' in which the midsection thereof is enlarged. Hence, by displaying the observation image as described above, it is possible to observe states of each of the observation areas. Further, the endoscope system of the present modification is effective for when the size of the display screen provided on the monitor 14, for example, is small so that it is difficult to enlarge and display the entire observation image.

As described above, in the endoscope system and the endoscope apparatus according to the present invention, although the imaging angle of view is wide when the wide angle endoscope observation image that is captured at the wide viewing angle is displayed on the monitor, each of the observation areas are displayed small. However, by enlarging the image displaying region of the monitor at which the wide angle observation image is displayed, the entire wide angle observation image is displayed large to improve visibility. Further, by extending and enlarging the image section in which the surrounding image sections of the wide angle observation image is excluded and by displaying the image section, visibility of the wide angle observation image can be improved.

As described above, an endoscope in an endoscope system, an endoscope apparatus, and an image processing apparatus according to the present invention are useful for an endoscope with a wide viewing angle.

### INDUSTRIAL APPLICABILITY

As described above, an endoscope system, an endoscope apparatus, and an image processing apparatus according to the present invention are useful for an endoscope having a wide viewing angle for a medical use, and specifically, the endoscope system, the endoscope apparatus, and the image processing apparatus are useful when plural endoscope apparatuses are used.

## Claims

1. An endoscope system comprising:
a first endoscope (11) that includes
a first insertion portion (11'; 45) having a first bendable portion (43) that is bendable over a first range,
a first distal end portion (44) including a first observation optical system, the first observation optical system having a first viewing angle (β₂) and an optical axis changing depending on bending of the first bendable portion (43);
a second endoscope (12) that includes
a second insertion portion (12'; 45) having a second bendable portion (43) that is bendable over a second range narrower than the first range, and
a second distal end portion (44) including a second observation optical system, the second observation optical system having a second viewing angle (β₁) and an optical axis changing depending on bending of the second bendable portion (43),
**characterized in that** the second viewing angle (β₁) is wider than the first viewing angle (β₂),
and further **characterized by** an image processing apparatus (13) including
a display size determining unit (29) configured to determine a display size of an observation image based on the viewing angle of the observation optical system that generates the observation image to be displayed so that a difference between a display size on a display screen of a region (14a) of a first observation image (19),
the first observation image (19) being acquired by the first endoscope (11) and the display size of a region (14a) of a second observation image (19'),
the second observation image (19') being acquired by the second endoscope (12) is reduced, wherein the display size determining unit (29) is configured to determine the display size so that a display size of the second observation image (19') becomes large compared to a display size of the first observation image (19); and
a size conversion processing unit (26) that is configured to electronically enlarge or contract the second observation image (19') so that the second observation image has the first display size.

2. The endoscope system according to claim 1, wherein
an angle of view of observation of the second endoscope (12) is wider than or equal to an angle of view of observation of the first endoscope (11) when a bending angle of the first bendable portion (43) is the same as a bending angle of the second bendable portion (43).

3. The endoscope system according to claim 1 or 2, wherein
an outside diameter (φ₃) of the second insertion portion (43) is larger than an outside diameter (φ₄) of the first insertion portion (43).

4. The endoscope system according to claim 1, wherein
the display size determining unit (29) is configured to determine the first display size so that the second display size becomes substantially the same as the third display size.

5. The image processing apparatus (13) according to claim 1 or 4, wherein
the size conversion processing unit (26) is configured to further clip out only a midsection (19c') of the second observation image (19') in order to display the second observation image enlarged with the first display size.

## Patentansprüche

1. Endoskopsystem, das aufweist:
ein erstes Endoskop (11) mit
einem ersten Einführbereich (11'; 45), der einen ersten Biegeabschnitt (43) aufweist, welcher über einen ersten Bereich biegsam ist,
einem ersten distalen Endbereich (44), der ein erstes optisches Beobachtungssystem aufweist, wobei das erste optische Beobachtungssystem einen ersten Sichtwinkel (β₂) und eine optische Achse besitzt, die sich in Abhängigkeit vom Biegen des ersten Biegeabschnitts (43) verändert;
ein zweites Endoskop (12) mit
einem zweiten Einführbereich (12'; 45), der einen zweiten Biegeabschnitt (43) aufweist, welcher über einen zweiten Bereich biegsam ist, der enger als der erste Bereich gefasst ist, und
einem zweiten distalen Endbereich (44), der ein zweites optisches Beobachtungssystem aufweist, wobei das zweite optische Beobachtungssystem einen zweiten Sichtwinkel (β₁) und eine optische Achse besitzt, die sich in Abhängigkeit vom Biegen des zweiten Biegeabschnitts (43) verändert;
**dadurch gekennzeichnet, dass** der zweiten Sichtwinkel (β₁) größer als der erste Sichtwinkel (β₂) ist,
und des Weiteren durch eine Bildverarbeitungsvorrichtung (13) gekennzeichnet, die umfasst:
eine Anzeigengröße-Bestimmungseinheit (29), die so ausgebildet ist, dass sie eine Anzeigengröße eines Beobachtungsbilds auf der Grundlage des Sichtwinkels des optischen Beobachtungssystems, welches das anzuzeigende Beobachtungsbild erzeugt, so bestimmt, dass auf einem Bildschirm eine Abweichung zwischen einer Anzeigengröße eines Bereichs (14a) eines ersten Beobachtungsbilds (19), wobei das erste Beobachtungsbild (19) durch das erste Endoskop (11) aufgenommen wird, und der Anzeigengröße eines Bereichs (14a) eines zweiten Beobachtungsbilds (19'), wobei das zweite Beobachtungsbild (19') durch das zweite Endoskop (12) aufgenommen wird, verringert wird, wobei die Anzeigengröße-Bestimmungseinheit (29) so ausgebildet ist, dass sie die Anzeigengröße so bestimmt, dass eine Anzeigengröße des zweiten Beobachtungsbilds (19') im Vergleich zu einer Anzeigengröße des ersten Beobachtungsbilds (19) groß wird; und
eine Größenumwandlungs-Verarbeitungseinheit (26), die so ausgebildet ist, dass sie das zweite Beobachtungsbild (19') elektronisch so vergrößert oder verkleinert, dass das zweite Beobachtungsbild die erste Anzeigengröße aufweist.

2. Endoskopsystem nach Anspruch 1, wobei
ein Beobachtungssichtwinkel des zweiten Endoskops (12) größer oder gleich einem Beobachtungssichtwinkel des ersten Endoskops (11) ist, wenn ein Biegewinkel des ersten Biegeabschnitts (43) gleich einem Biegewinkel des zweiten Biegeabschnitts (43) ist.

3. Endoskopsystem nach Anspruch 1 oder 2, wobei
ein Außendurchmesser (Φ₃) des zweiten Einführbereichs (43) größer als ein Außendurchmesser (Φ₄) des ersten Einführbereichs (43) ist.

4. Endoskopsystem nach Anspruch 1, wobei
die Anzeigengröße-Bestimmungseinheit (29) so ausgebildet ist, dass sie die erste Anzeigengröße so bestimmt, dass sich die zweite Anzeigengröße im Wesentlichen der dritten Anzeigengröße angleicht.

5. Bildverarbeitungsvorrichtung (13) nach Anspruch 1 oder 4, wobei
die Größenumwandlungs-Verarbeitungseinheit (26) so ausgebildet ist, dass sie ferner nur einen Mittelteil (19c') des zweiten Beobachtungsbilds (19') ausschneidet, um das zweite Beobachtungsbild mit der ersten Anzeigengröße vergrößert anzuzeigen.

## Revendications

1. Système d'endoscope comprenant :
un premier endoscope (11) qui comprend
une première partie d'insertion (11' ; 45) comportant une première partie cintrable (43) qui peut être cintrée sur une première plage,
une première partie d'extrémité distale (44) comprenant un premier système optique d'observation, le premier système optique d'observation comportant un premier angle de vision (β₂) et un axe optique changeant en fonction du cintrage de la première partie cintrable (43) ;
un deuxième endoscope (12) qui comprend
une deuxième partie d'insertion (12' ; 45) comportant une deuxième partie cintrable (43) qui peut être cintrée sur une deuxième plage plus étroite que la première plage, et
une deuxième partie d'extrémité distale (44) comprenant un deuxième système optique d'observation, le deuxième système optique d'observation comportant un deuxième angle de vision (β₁) et un axe optique changeant en fonction du cintrage de la deuxième partie cintrable (43),
**caractérisé en ce que** le deuxième angle de vision (β₁) est plus large que le premier angle de vision (β₂),
en outre **caractérisé par** un appareil de traitement d'images (13) comprenant :
une unité de détermination de taille d'affichage (29) configurée pour déterminer une taille d'affichage d'une image d'observation sur la base de l'angle de vision du système optique d'observation qui génère l'image d'observation devant être affichée de sorte qu'une différence entre une taille d'affichage sur un écran d'affichage d'une région (14a) d'une première image d'observation (19), la première image d'observation (19) étant obtenue par le premier endoscope (11) et la taille d'affichage d'une région (14a) d'une deuxième image d'observation (19'), la deuxième image d'observation (19') étant obtenue par le deuxième endoscope (12) est réduite, dans lequel l'unité de détermination de taille d'affichage (29) est configurée pour déterminer la taille d'affichage de sorte qu'une taille d'affichage de la deuxième image d'observation (19') devient grande comparée à une taille d'affichage de la première image d'observation (19) ; et
une unité de traitement de conversion de taille (26) qui est configurée pour agrandir ou rétrécir de façon électronique la deuxième image d'observation (19') de sorte que la deuxième image d'observation présente la première taille d'affichage.

2. Système d'endoscope selon la revendication 1, dans lequel
un angle de vue d'observation du deuxième endoscope (12) est plus large ou égal à un angle de vue d'observation du premier endoscope (11) lorsqu'un angle de cintrage de la première partie cintrable (43) est le même qu'un angle de cintrage de la deuxième partie cintrable (43).

3. Système d'endoscope selon la revendication 1 ou 2, dans lequel
un diamètre extérieur (φ₃) de la deuxième partie d'insertion (45) est plus large qu'un diamètre extérieur (φ4) de la première partie d'insertion (45).

4. Système d'endoscope selon la revendication 1, dans lequel
l'unité de détermination de taille d'affichage (29) est configurée pour déterminer la première taille d'affichage de sorte que la deuxième taille d'affichage devient sensiblement la même que la troisième taille d'affichage.

5. Appareil de traitement d'images (13) selon la revendication 1 ou 4, dans lequel
l'unité de traitement de conversion de taille (26) est configurée pour en outre extraire seulement une section intermédiaire (19c') de la deuxième image d'observation (19') afin d'afficher la deuxième image d'observation agrandie avec la première taille d'affichage.
